(19) Europäisches Patentamt
European Patent Office
Office européen des brevets

(11) Veröffentlichungsnummer: **0 141 054**
**B1**

# (12) EUROPÄISCHE PATENTSCHRIFT

(45) Veröffentlichungstag der Patentschrift: **02.07.86**

(51) Int. Cl.⁴: **C 07 C 35/08**, C 07 C 29/20

(21) Anmeldenummer: **84108733.1**

(22) Anmeldetag: **24.07.84**

(54) **Verfahren zur Herstellung von 2- und 4-tert.-Butylcyclohexanol mit hohen Anteilen an cis-Isomeren durch katalytische Hydrierung der entsprechenden tert.-Butylphenole.**

(30) Priorität: **22.10.83 DE 3338437**
**17.01.84 DE 3401343**

(43) Veröffentlichungstag der Anmeldung:
**15.05.85 Patentblatt 85/20**

(45) Bekanntmachung des Hinweises auf die Patenterteilung:
**02.07.86 Patentblatt 86/27**

(84) Benannte Vertragsstaaten:
**BE CH DE FR GB IT LI NL**

(56) Entgegenhaltungen:
**CH-A-590 186**
**DE-A-2 909 663**
**DE-B-1 102 731**

**PATENT ABSTRACTS OF JAPAN, unexamined applications, C Sektion, Band 3, Nr. 114, 21. September 1979, THE PATENT OFFICE JAPANESE GOVERNMENT, Seite 125 C 59**

(73) Patentinhaber: **HÜLS AKTIENGESELLSCHAFT, Patentabteilung / PB 15 - Postfach 13 20, D-4370 Marl 1 (DE)**

(72) Erfinder: **Otte, Werner, Dr., Eichenstück 55, D-4270 Dorsten 11 (DE)**
Erfinder: **Nehring, Rudolf Dr., Griesheimer Strasse 12, D-4370 Marl (DE)**
Erfinder: **zur Hausen, Manfred, Dr., Hoechster Strasse 1, D-4370 Marl (DE)**

EP 0 141 054 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Herstellung von 2- und 4-tert.-Butylcyclohexanolen durch zweistufige katalytische Hydrierung der entsprechenden tert.-Butylphenole, wobei man das cis-Isomere in hohen Anteilen im Hydrieraustrag erhält.

Bisher war es nur möglich, beide Produkte mit erhöhtem cis-Isomerenanteil diskontinuierlich in Lösung zu gewinnen. Dies erfordert jedoch eine zusätzliche Reindestillationsstufe (GB-PS 2 016 449 = DE-OS 29 09 663, DE-PS 21 32 547).

Da die 2- und 4-tert.-Butylcyclohexanole mit hohen Anteilen an cis-Isomeren von größer werdendem wirtschaltlichen Interesse sind, bestand die Aufgabe, ein Verfahren zu finden, das es gestattet, beide Produkte mit erhöhten Anteilen an cis-Isomeren in einfacher und wirtschaftlicher Weise herzustellen.

Diese Aufgabe wird erfindungsgemäß entsprechend den Angaben der Patentansprüche gelöst.

Überraschenderweise erhält man bei dem erfindungsgemäßen Verfahren ohne Zusatz von Lösemitteln und ohne nachfolgende Reinigung die gewünschten 2- und 4-tert.-Butylcyclohexanole mit hohen cis-Anteilen in hoher Ausbeute und Reinheit. Besonders hohe cis-Anteile, bis 90 %, erhält man beim 2-tert.-Butylcyclohexanol. Ein weiterer Vorteil des erfindungsgemäßen Verfahrens ist die kontinuierliche Durchführung der Hydrierung. Die so hergestellten cis-reichen tert.-Butylcyclohexanole können unmittelbar für die Weiterverarbeitung, insbesondere für die Herstellung vom Duftstoffen oder Duftstoffprodukten eingesetzt werden.

Die 2- und 4-tert.-Butylphenole werden zunächst in einer ersten Stufe mit Wasserstoff über einen festangeordneten Pd-Kontakt, beispielsweise 0,1 bis 5 Gew.-%, vorzugsweise 0,5 bis 1 Gew.-% Pd auf einem inerten Träger, z. B. $Al_2O_3$, bevorzugt in der Rieselphase, bei Temperaturen von 120 bis 280 °C, vorzugsweise 160 bis 220 °C und Drücken über 200 bar, vorzugsweise 250 bis 350 bar, geleitet, wobei man bevorzugt einen Teil des Hydrieraustrages dem Einsatzstoff vor dem Reaktor zuführt. Der Anteil das rückgeführten Hydrieraustrages der ersten Stufe beträgt die 1- bis 20 fache, vorzugsweise die 5- bis 10 fache Menge des Rohproduktzulaufs. Der Umsatz an tert.-Butylphenolen liegt in dieser ersten Stufe bei 65 bis 98 %, vorzugsweise bei 80 bis 90 %.

Den nicht rückgeführten Teil des Hydrieraustrages mit den Restgehalten an tert.-Butylphenolen und tert.-Butylcyclohexanon als Zwischenprodukt hydriert man in einer zweiten Stufe an einem fest angeordneten Ru-Kontakt, beispielsweise 0,1 bis 5 %, vorzugsweise 0,5 bis 1 % Ru auf einem inerten Träger, z. B. $Al_2O_3$, bevorzugt in der Rieselphase, ohne Rückvermischung bei Temperaturen von 70 bis 200 °C, vorzugsweise von 100 bis 150 °C und Drücken über 200 bar, vorzugsweise von 250 bis 350 bar aromatenfrei. Die Aromatengehalte liegen unter 0,2 %, vorzugsweise unter 10 ppm.

Im allgemeinen arbeitet man bei Zulaufmengen von 0,1 bis 5 l Zulauf/l Katalysator. h.

Eine zweistufige Hydrierung, bei der man in beiden Stufen einen Pd-Kontakt einsetzt, ermöglicht nicht die Herstellung eines tert.-Butylcyclohexanols mit hohen cis-Anteilen, wie das Vergleichsbeispiel A zeigt.

Bei einem sehr niedrigen Aromatengehalt enthält der Hydrieraustrag immer noch erhebliche Mengen an tert.-Butylcyclohexanon (44 bis 55 %), die in einer weiteren Hydrierung (3. Stufe) z. B. an CuCr-Kontakten zu den tert.-Butylcyclohexanolen hydriert werden müßten, wodurch das Endprodukt erheblich verteuert wird.

Eine einstufige Hydrierung an Ru-Katalysatoren erfordert so scharfe Hydrierbedingungen, daß der Anteil an cis-Isomeren gegenüber den nach dem erfindungsgsmäßen Verfahren herstellbaren deutlich abfällt. Bei weniger scharfen Bedingungen ist eine Nachhydrierung erforderlich (Vergleichsbeispiele B und C). Wie die Vergleichsbeispiele B und C zeigen, ist zudem beim Einsatz des Ru-Kontaktes der Durchsatz wesentlich geringer als beim Pd-Kontakt.

Man erhält die tert.-Butylcyclohexanole mit hohem cis-Gehalt in hoher Reinheit, so daß sie ohne weitere Reinigung zur Herstellung von Duftstoffen eingesetzt werden können.

**Beispiel 1**

27 l 2-tert.-Butylphenol (2-TBPHOL) leitet man mit Wasserstoff bei 200 °C und 300 bar Gesamtdruck stündlich durch einen Durchflußreaktor (Volumen 20 l), der mit 20 l eines Pd-Katalysators (0,5 Gew.-% Pd auf $Al_2O_3$) gefüllt ist. 250 l/h des Hydrieraustrages mischt man mit dem Zulauf und führt das Gemisch durch den Reaktor. Der Hydrieraustrag hat im Mittel folgende Zusammensetzung:

0,3 % tert.-Butylcyclohexan (TBCAN)
60 % 2-tert.-Butylcyclohexanon (2-TBCON)
18 % cis-2-tert.-Butylcyclohexanol (cis-2-TBCOL)
5 % trans-2-tert.-Butylcyclohexanol (tr-2-TBCOL)
17 % 2-tert.-Butylphenol (2-TBPHOL)

400 ml dieses Hydrieaustrages führt man stündlich mit Wasserstoff bei 140 °C und 300 bar Gesamtdruck durch einen zweiten Durchflußreaktor (Volumen 400 ml), der mit 400 ml eines Ru-Kontaktes (0,5 Gew.-% Ru auf $Al_2O_3$) gefüllt ist.

Der Hydrieraustrag hat folgende Zusammensetzung:

0,2 % tert.-Butylcyclohexan (TBCAN)
0,3 % 2-tert.-Butylcyclohexanon (2-TBCON)

85,1 % cis-2-tert.-Butylcyclohexanol (cis-2-TBCOL)
14,4 % trans-2-tert.-Butylcyclohexanol (tr-2-TBCOL)
<10 ppm 2-tert.-Butylphenol (2-TBPHOL)

**Beispiel 2**

Man arbeitet nach den Angaben des Beispiels 1, führt jedoch 40 l/h statt 27 l/h 2-TBPHOL bei 220 °C statt 200 °C durch den ersten Reaktor.

Der Hydrieraustrag hat folgende Zusammensetzung:
0,1 % TBCAN
58,2 % 2-TBCON
8,7 % cis-2-TBCOL
2,7 % tr-2-TBCOL
30,3 % 2-TBPHOL

400 ml dieses Hydrieraustrages der 1. Stufe werden stündlich nach den Angaben des Beispiels 1 in der zweiten Stufe hydriert.

Der Hydrieraustrag hat folgende Zusammensetzung:
0,1 % TBCAN
0,2 % 2-TBCON
83,4 % cis-2-TBCOL
16,3 % tr-2-TBCOL
<10 ppm 2-TBPHOL

**Beispiel 3**

Nach den Angaben des Beispiels 1 hydriert man in 1. Stufe 2-TBPHOL jedoch 17 l/h 2-TBPHOL mit 200 l/h Produktrückführung bei 160 °C mit folgendem Ergebnis:
76,8 % 2-TBCON
9,3 % cis-2-TBCOL
2,4 % tr-2-TBCOL
11,5 % 2-TBPHOL

Die Hydrierung in der 2. Stufe erfolgt nach den Angaben des Beispiels 1 mit vergleichbarem Ergebnis.

**Beispiel 4**

Man hydriert 2-TBPHOL nach den Angaben des Beispiels 3, jedoch bei 170 °C in der ersten Stufe, mit folgendem Ergebnis:
75,8 % 2-TBCON
15,4 % cis-2-TBCOL
4,0 % tr-2-TBCOL
4,8 % 2-TBPHOL

Die Hydrierung in der 2. Stufe erfolgt nach den Angaben des Beispiels 1 mit vergleichbarem Ergebnis.

**Beispiel 5**

Man hydriert 2-TBPHOL nach den Angaben des Beispiels 3, jedoch bei 180 °C in der ersten Stufe, mit folgendem Ergebnis:
67,1 % 2-TBCON
24,9 % cis-2-TBCOL
6,4 % tr-2-TBCOL
1,6 % 2-TBPHOL

Die Hydrierung in der 2. Stufe erfolgt nach den Angaben des Beispiels 1 mit vergleichbarem Ergebnis.

**Vergleichsbeispiel A**

Man hydriert in der ersten Stufe 2-TBPHOL nach den Angaben des Beispiels 1. Im zweiten Reaktor hydriert man den Hydrieraustrag der ersten Stufe bei 150 °C über 400 ml des Pd-Kontaktes (0,5 % Pd auf $Al_2O_3$).

Im Mittel hat der Hydrieraustrag der 2. Stufe folgende Zusammensetzung:

0,3 % TBCAN
44,3 % 2-TBCON
45,7 % cis-2-TBCOL
9,7 % tr-2-TBCOL
<10 ppm 2-TBPHOL

Erhöht man in der zweiten Stufe die Temperatur von 150 °C auf 200 °C bis kein 2-TBCON mehr erkennbar ist, so hat der Hydrieraustrag der zweiten Stufe folgende Zusammensetzung:

1,8 % 2-TBCAN
<0,1 % 2-TBCON
62,7 % cis-2-TBCOL
33,9 % tr-2-TBCOL
95 ppm 2-TBPHOL
1,6 % unbekannte Verbindungen

**Beispiel 6**

Die Hydrierung von 2-TBPHOL erfolgt in der ersten Stufe nach den Angaben des Beispiels 1. In der zweiten Stufe setzt man einen Ru-Katalysator ein, der 1 % Ru auf $Al_2O_3$ emthält. Man hydriert an diesem Ru-Katalysator bei 120 °C 160 ml/h des nach Beispiel 1, Stufe 1 erhaltenen Hydrieraustrages. Der Hydrieraustrag der 2. Stufe hat im Mittel folgende Zusammensetzung:

0,3 % TBCAN
81,7 % cis-2-TBCOL
18,0 % tr-2-TBCOL
<0,1 % 2-TBPHOL

**Beispiel 7**

Man hydriert nach den Angaben des Beispiels 3, jedoch bei 160 °C mit einem Zulauf von 21 l/h 2-TBPHOL und einer Rückführung von 275 l/h Hydrieraustrag mit folgendem Ergebnis:

0,4 % TBCAN
78,1 % 2-TBCON
6,7 % cis-2-TBCOL
1,9 % tr-2-TBCOL
12,9 % 2-TBPHOL

Die Hydrierung in der 2. Stufe erfolgt nach den Angaben des Belspiels 1 mit vergleichbarem Ergebnis.

**Beispiel 8**

Die Hydrierung erfolgt nach den Angaben des Beispiels 7, jedoch werden 150 l/h Hydrieraustrag zurückgeführt. Man erhält in der 1. Stufe einen Hydrieraustrag folgender Zusammensetzung:

0,2 % TBCAN
76,1 % 2-TBCON
6,3 % cis-2-TBCOL
1,8 % tr-2-TBCOL
15,6 % 2-TBPHOL

Die Hydrierung in der 2. Stufe erfolgt nach den Angaben des Beispiels 1 mit vergleichbarem Ergebnis.

**Beispiel 9**

Die Hydrierung der ersten Stufe erfolgt nach den Angahen des Beispiels 1. In der zweiten Stufe hydriert man bei 100 °C 200 ml/h des in der 1. Stufe erhaltenen Hydrieraustrages an dem im Beispiele 6 eingesetzten Ru-Kontakt (1 % Ru auf $Al_2O_3$). Man erhält einen Hydrieraustrag folgender Zusammensetzung:

0,3 % 2-TBCON
80,8 % cis-2-TBCOL
18,9 % tr-2-TBCOL
<0,1 % 2-TBPHOL

**Vergleichsbeispiel B**

Durch den in Beispiel 1 in der zweiten Stufe eingesetzten 400 ml Durchflußreaktor, der mit 400 ml des in Beispiel 6 eingesetzten Ru-Kontaktes (1 % auf $Al_2O_3$) gefüllt ist, leitet man mit Wasserstoff stündlich 400 ml reines 2-TBPHOL (also 1. Stufe) bei 100 °C und 300 bar. Der Hydrieraustrag hat im Mittel folgende Zusammensetzung:
14,2 % 2-TBCON
74,4 % cis-2-TBCOL
11,2 % tr-2-TBCOL
0,2 % 2-TBPHOL

**Vergleichsbeispiel C**

Führt man die Hydrierung nach den Angaben des Vergleichsbeispiels B, jedoch bei 180 °C durch, so erhält man folgende Ergebnisse:
0,6 % TBCAN
75,1 % cis-2-TBCOL
24,3 % tr-2-TBCOL
<10 ppm 2-TBPHOL

**Beispiel 10**

Durch den im Beispiel 1 (2. Stufe) beschriebenen 400 ml-Reaktor, der mit 400 ml des im Beispiel 1 angegebenen Pd-Kontaktes gefüllt ist, leitet man mit Wasserstoff stündlich 80 ml reines 2-TBPHOL (also 1. Stufe, ohne Rückvermischung) bei 120 °C und 300 bar. Der Hydrieraustrag hat im Mittel folgende Zusammensetzung:
64,1 % 2-TBCON
27,0 % cis-2-TBCOL
6,0 % tr-2-TBCOL
2,9 % 2-TBPHOL
Die Hydrierung in der 2. Stufe erfolgt nach den Angaben des Beispiels 1 mit vergleichbarem Ergebnis.

**Beispiel 11**

Durchführung wie Beispiel 1, jedoch wurde ein Katalysator mit 0,1 % Pd auf $Al_2O_3$ eingesetzt. Der Zulauf an o-TBPHOL beträgt bei 200 °C und 300 bar Gesamtdruck 20 l/h, der mit 200 l/h Hydrieraustrag vermischt wird. Dieser Hydrieraustrag hat im Mittel folgende Zusammensetzung:
0,1 % 2-TBCAN
72,3 % 2-TBCON
16,8 % cis-2-TBCOL
3,3 % tr-2-TBCOL
7,5 % 2-TBPHOL
Die Hydrierung in der 2. Stufe erfolgt nach den Angaben des Beispiels 1 mit vergleichbarem Ergebnis.

**Beispiel 12**

Der Hydrieraustrag der 1. Stufe nach Beispiel 1 wird in der im Beispiel 1 genannten 400 ml-Apparatur (2. Stufe) an einem Katalysator hydriert, der 0,1 % Ru auf $Al_2O_3$ enthält. Bei 120 °C, 300 bar und einem Zulauf von 80 ml/h hat das Endprodukt folgende Zusammensetzung:
0,1 % TBCAN

80,4 % cis-2-TBCOL
19,5 % tr-2-TBCOL
<10 ppm 2-TBPHOL

**Vergleichsbeispiel D**

Führt man die Hydrierung nach den Angaben des Beispiels 1, jedoch mit 4-tert.-Butylphenol (4-TBPHOL) statt mit 2-tert.-Butylphenol, bei einer Temperatur von 195 °C statt 200 °C und einem Zulauf von 10 l/h statt 27 l/h sowie einer Rückvermischung von 150 l/h statt 250 l/h durch, so erhält man in der ersten Stufe einen Hydrieraustrag folgender Zusammensetzung:
0,2 % 4-TBCAN
4,0 % 4-TBCON
28,1 % cis-4-TBCOL
65,4 % trans-4-TBCOL
2,1 % 4-TBPHOL
0,2 % unbekannte Verbindungen

Die zweite Stufe führt man nach den Angaben des Beispiels 1 durch, hydriert jedoch den Hydrieraustrag der ersten Stufe (400 ml/h) statt an dem dort angegebenen Ru-Kontakt an dem gleichen Pd-Kontakt, wie er in der ersten Stufe eingesetzt ist, bei einer Temperatur von 210 °C statt 140 °C.

Der Hydrieraustrag der zweiten Stufe hat folgende Zusammensetzung:
0,2 % 4-TBCAN
<0,1 % 4-TBCON
29,3 % cis-4-TBCOL
70,3 % trans-4-TBCOL
<0,01 % 4-TBPHOL
0,2 % unbekannte Verbindungen

**Beispiel 13**

Man arbeitet nach den Angaben des Beispiels 1, setzt jedoch 4-tert.-Butylphenol (4-TBPHOL) statt 2-tert.-Butylphenol ein und hydriert in der ersten Stufe bei einem Zulauf von 8 l/h statt 27 l/h und bei einer Temperatur von 150 °C statt 200 °C. Der Hydrieraustrag der ersten Stufe hat folgende Zusammensetzung:
10,8 % 4-TBCON
30,7 % cis-4-TBCOL
27,4 % trans-4-TBCOL
30,7 % 4-TBPHOL
0,4 % unbekannte Verbindungen

Die zweite Hydrierstufe führt man nach den Angaben des Beispiels 1 durch, jedoch bei einem Zulauf von 120 ml/h des Hydrieraustrages der 1. Stufe statt 400 ml und bei einer Temperatur von 150 °C statt 140 °C.

Der Hydrieraustrag der zweiten Stufe hat im Mittel folgende Zusammensetzung:
0,2 % 4-TBCAN
- % 4-TBCON
46,4 % cis-4-TBCOL
53,1 % trans-4-TBCOL
<0,01 % 4-TBPHOL
0,3 % unbekannte Verbindungen

**Patentansprüche**

1. Verfahren zur Herstellung von 2- und 4-tert.-Butylcyclohexanolen mit hohen Anteilen an cis-Isomeren der allgemeinen Formel

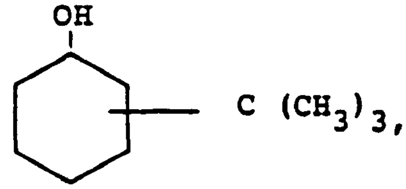

ausgenommen 3-tert.-Butylcyclohexanol, durch Hydrierung der entsprechenden tert.-Butylphenole mit Wasserstoff in Gegenwart von Pd- und Ru-Katalysatoren,
dadurch gekennzeichnet,
daß man die Hydrierung kontinuierlich in Abwesenheit eines Lösemittels zweistufig bei Wasserstoffdrücken über 200 bar durchführt und in der ersten Stufe an einem Pd-Kontakt bei Temperaturen von 120 bis 280 °C und in der zweiten Stufe an einem Ru-Kontakt bei Temperaturen von 70 bis 200 °C hydriert.
2. Verfahren nach Anspruch 1,
dadurch gekennzeichnet,
daß man in der ersten Stufe bei einer Temperatur von 160 bis 220 °C und in der zweiten Stufe bei einer Temperatur von 100 bis 150 °C hydriert.
3. Verfahren nach den Ansprüchen 1 und 2,
dadurch gekennzeichnet,
daß man bei einem Druck von 250 bis 350 bar hydriert.
4.Verfahren nach den Ansprüchen 1 bis 3,
dadurch gekennzeichnet,
daß man die Hydrierung in der ersten Stufe mit Produktrückvermischung durchführt, wobei die rückgeführte Menge das 1- bis 20 fache, vorzugsweise das 5- bis 10 fache der Zulaufmenge beträgt.
5. Verfahren nach den Ansprüchen 1 bis 4,
dadurch gekennzeichnet,
daß man als Pd- und/oder Ru-Kontakte Pd- bzw. Ru-Trägerkontakte einsetzt, in denen das Pd bzw. Ru auf inerten Trägern, vorzugsweise auf Aluminiumoxid, aufgebracht ist.
6. Verfahren nach den Ansprüchen 1 bis 5,
dadurch gekennzeichnet,
daß man das Pd bzw. Ru aut dem Träger in Konzentrationen von 0,1 bis 5 Gew.-%, vorzugsweise von 0,5 bis 1 Gew.-% einsetzt.

**Claims**

1. A process for the production of 2- and 4-tert.-butylcyclohexanol with a high proportion of cis-isomers of the general formula

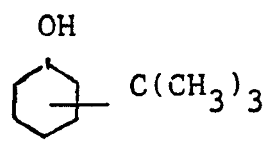

other than 3-tert.-butylcyclohexanol, by hydrogenation of the corresponding tert.-butylphenols with hydrogen in the presence of Pd and Ru catalysts, characterised in that the hydrogenation is carried out continuously in two stages at a hydrogen pressure in excess of 200 bar in the absence of a solvent, a Pd contact catalyst and a tempeature of 120 to 280°C being used in the first stage and a Ru contact catalyst and a temperature of 70 to 200°C being used in the second stage.
2. A process according to claim 1, characterised in that the hydrogenation is carried out at 160 to 220°C in the first stage and 100 to 150°C in the second stage.
3. A process according to claim 1 or 2, characterised in that the hydrogenation is carried out at a pressure of 250 to 350 bar.
4. A process according to any of claims 1 to 3, characterised in that the hydrogenation is carried out in the first stage on a product recycle mixture, the recycled quantity being 1 to 20 times, preferably 5 to 10 times, the quantity of material passing to the search stage.
5. A process according to any of claims 1 to 4, characterised in that the Pd and/or Ru contact catalyst is a supported contact catalyst in which Pd and/or Ru is supported on an inert support, preferably aluminium oxide.
6. A process according to any of claims 1 to 5, characterised in that the Pd and/or Ru is introduced onto the carrier in a concentration of 0.1 to 5% by weight, preferably 0.5 to 1% by weight.

**Revendications**

1. Procédé pour la préparation de 2- et 4-tertio-butyl-cyclohexanols à fortes proportions d'isomères cis, de la formule générale :

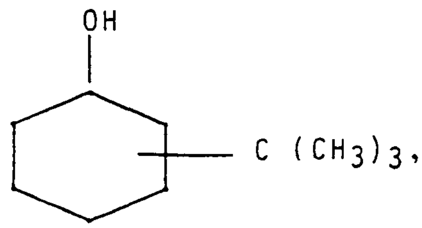

à l'exception du 3-tertio-butyl-cyclohexanol, par hydrogénation des tertio-butyl-phénols correspondants par l'hydrogène en présence de catalyseurs Pd et Ru, caractérisé par le fait que l'on conduit l'hydrogénation de façon continue en l'absence de solvant, en deux étapes à des pressions d'hydrogène supérieures à 200 bar et que l'on hydrogène à la première étape sur un catalyseur Pd à des températures de 120 à 280°C et à la deuxième étape sur un catalyseur Ru à des températures de 70 à 200°C.

2. Procédé selon la revendication 1, caractérisé par le fait que l'on hydrogène à la première étape à une température de 160 à 220°C et à la deuxième étape à une température de 100 à 150°C.

3. Procédé selon les revendications 1 et 2, caractérisé par le fait que l'on hydrogène à une pression de 250 à 350 bar.

4. Procédé selon les revendications 1 à 3, caractérisé par le fait que l'on conduit l'hydrogénation à la première étape en mélangeant à nouveau le produit, la quantité recyclée représentant de 1 à 20 fois, de préférence de 5 à 10 fois la quantité amenée.

5. Procédé selon les revendications 1 à 4, caractérisé par le fait que l'on utilise, comme catalyseurs Pd et/ou Ru, des catalyseurs Pd ou Ru sur support, dans lesquels le Pd ou le Ru est appliqué sur des supports inertes, de préférence sur de l'oxyde d'aluminium.

6. Procédé selon les revendications 1 à 5, caractérisé par le fait que l'on utilise le Pd ou le Ru sur le support à des concentrations de 0,1 à 5 % en poids, de préférence de 0,5 à 1 % en poids.